# EUROPÄISCHE PATENTSCHRIFT

(11) **EP 2 355 794 B1**
(45) Veröffentlichungstag und Bekanntmachung des Hinweises auf die Patenterteilung: **21.08.2019**
(21) Anmeldenummer: 09764233.4
(22) Anmeldetag: 27.11.2009
(51) Int. Cl.: A61K 9/00, A61K 9/51, A61K 47/10, A61K 47/26

(54) **LÖSUNGEN VON LIPOPHILEN SUBSTANZEN, INSBESONDERE ARZNEISTOFFLÖSUNGEN**
SOLUTION OF LIPOPHILIC SUBSTANCES, ESPECIALLY MEDICINAL SOLUTIONS
SOLUTIONS DE SUBSTANCES LIPOPHILES, EN PARTICULIER SOLUTIONS DE MÉDICAMENT

(30) Priorität: 27.11.2008 DE 102008059201
(43) Veröffentlichungstag der Anmeldung: 17.08.2011
(73) Patentinhaber: Universität Regensburg, 93053 Regensburg (DE)
(72) Erfinder: GÖPFERICH, Achim, 93161 Sinzing (DE); LUSCHMANN, Christoph, 92287 Schmidmühlen (DE)
(74) Vertreter: Ahrens, Gabriele
(86) Internationale Anmeldenummer: PCT/EP2009/065982
(87) Internationale Veröffentlichungsnummer: WO 2010/060989

(56) Entgegenhaltungen:
- WO-A1-02/05815
- WO-A1-2006/133510
- WO-A2-99/08684
- US-A- 4 000 263
- US-A- 5 192 535
- BOWEN P: "Particle Size Distribution Measurement from Millimeters to Nanometers and from Rods to Platelets", JOURNAL OF DISPERSION SCIENCE AND TECHNOLOGY, TAYLOR AND FRANCIS GROUP, NEW YORK, NY, US, vol. 23, no. 5, 1 January 2002 (2002-01-01), pages 631-662, XP009102859, ISSN: 0193-2691, DOI: 10.1081/DIS-120015368

## Beschreibung

Gegenstand der vorliegenden Erfindung sind insbesondere Arzneimittelformulierungen für lipophile, schwer wasserlösliche Wirkstoffe, vorzugsweise Arzneistoffe. Das System ist für die topische Applikation insbesondere am Auge ganz besonders geeignet.

Für zahlreiche Erkrankungen stehen hochpotente Wirkstoffe bereit. Häufig sind diese aber sehr lipophil und damit schlecht wasserlöslich. Diese Eigenschaft der geringen Löslichkeit lässt sich nach dem europäischen Arzneibuch definieren über die Massenanteile an Lösungsmittel, die für die Herstellung einer Lösung von einem Massenteil Substanz benötigt wird (Europäisches Arzneibuch 5.0/1.04.00.00). In diesem Zusammenhang unterscheidet man:
- schwer löslich
   *pro Teil Stoff werden 100 bis 1000 Teile Lösungsmittel benötigt*
- sehr schwer löslich
   *pro Teil Stoff werden 1000 bis 10000 Teile Lösungsmittel benötigt*
- praktisch unlöslich
   *pro Teil Stoff werden über 10000 Teile Lösungsmittel benötigt.*

Es sind zahlreiche Vorschläge zur Löslichmachung und Verabreichung schwerlöslicher lipophiler pharmazeutischer Wirkstoffe bekannt.

So finden sich Vorschläge zur Herstellung von stabilen Lösungen der betreffenden Wirkstoffe für die orale Verabreichung, wobei durch Verwendung ausgewählter Lösungsmittelsysteme und durch Zusatz von Lösungsvermittlern der Wirkstoff stabil in Lösung gehalten werden soll. Beispiele hierfür finden sich in DE 40 03 844 A1, EP 0 488 181 A1, EP 0 650 730 A1, WO 95/29677 A1 und WO 99/08684 A2. DE 40 03 844 A1 betrifft insbesondere die Vermeidung von Ethanol für die Herstellung einer flüssigen pharmazeutischen Zusammensetzung enthaltend Cyclosporin als Wirkstoff.

EP 0 488 181 A1, EP 0 650 730 A1 sowie WO 95/29677 A1 betreffen speziell auf bestimmte Wirkstoffe wie Nifedipin, Rapamycin bzw. Camptothecin zugeschnittene Lösungsmittelsysteme. Gemäß WO 95/29677 A1 kann die erhaltende stabile Lösung auch parenteral injiziert werden.

Die hier beschriebenen Lösungsmittelsysteme können unter anderem Polyethylenglykol als Cosolvens oder als Lösungsverstärker enthalten. Ziel ist es den Wirkstoff stabil in Lösung zu halten, eine Präzipitation des Wirkstoffes am Applikationsort ist nicht vorgesehen.

In DE 694 02 022 T2 wird eine stabilisierte Arzneimittellösung für die parenterale Applikation von Wirkstoffen beschrieben, die einem durch Carboxylationen katalysierten Abbau unterliegen. Zur Löslichmachung wird eine Mischung aus einem Lösungsmittel und einem nichtionischen Lösungsvermittler vorgeschlagen, wobei als Lösungsmittel Polyethylenglykol eingesetzt werden kann. Durch den Lösungsvermittler wird sichergestellt, dass eine stabile Lösung erhalten wird, eine Ausfällung des Wirkstoffes ist nicht erwünscht.

US 4,000,263 beschreibt ein lagerfähiges flüssiges Mittel zur Behandlung von Akne, wobei Erythromycin als Wirkstoff in einem Lösungsmittelgemisch aus Propylenglycol, Ethylalkohol und einem ethoxylierten Ether von Laurylalkohol vorliegt. Die zu behandelnde Stelle wird mit der Lösung eingerieben, wobei der Wirkstoff nach Verdunsten der leichtflüchtigen Bestandteile auf der Stelle verbleibt.

WO 2006/133510 A1 betrifft eine Formulierung zur parenteralen Verabreichung von Docetaxel oder eines pharmazeutisch wirksamen Salzes davon. Auch hier ist das zum Einsatz kommende Lösungsmittelgemisch und dessen Zusätze derart ausgewählt, dass der Wirkstoff stabil in Lösung verbleibt. Eine Ausfällung ist nicht erwünscht.

WO 2008/101344 A1 betrifft eine Zusammensetzung, die lipophile bioaktive Verbindungen, wie insbesondere Coenzym Q10, gelöst enthält. Die Zusammensetzung kann für medizinische, kosmetische oder für Nahrungsmittelanwendungen eingesetzt werden. Der Wirkstoff wird mit einem nichtionischen Emulgator vermischt und geschmolzen, und die Schmelze in Wasser unter Ausbildung einer klaren Lösung eingerührt. Polyethylenglykol wird hier als Stabilisator zur Lyophilisation, nicht aber als Lösungsmittel eingesetzt.

Das US Patent 4,000,263 A betrifft eine lagerstabile Lösung enthaltend Erythromycin, Propylenglycol, Ethylalkohol und einen ethoxylierten Ether von Laurylalkohol. Diese Lösung wird zur Behandlung von Akne eingesetzt und dafür auf die Haut aufgetragen.

WO 99/08684 betrifft eine pharmazeutische Lösung enthaltend ein Azasteroid, Polyethylenglycol und Propylenglycol. Insbesondere betrifft diese Druckschrift Gelatinekapseln, die mit dieser Lösung gefüllt sind. Nach dieser Druckschrift kann die pharmazeutische Lösung in Form der Lösung, als Gelatinekapsel oder als Tablette verabreicht werden. Es findet sich jedoch kein Hinweis auf die Verabreichung am Auge noch auf die Ausbildung einer Nanosuspension bei Kontakt mit einer wässrigen Körperflüssigkeit.

Problematisch bei lipophilen, schwer bis praktisch unlöslichen Substanzen im Rahmen einer Therapie ist die geringe Verfügbarkeit des Wirkstoffs am Wirkort in gelöster Form. Die ist von herausragender Bedeutung, da in der Regel lediglich die im wässrigen biologischen Milieu gelöste Substanz in der Lage ist an ihre Zielstruktur, wie beispielsweise Rezeptoren an der Zelloberfläche zu gelangen.

Darüber hinaus ergeben sich Probleme bezüglich des Transports schlecht löslicher Stoffe über biologische Barrieren wie der Darmwand nach oraler Applikation oder der Hornhaut bei Applikation am Auge, da in der Regel lediglich die gelöste Substanz in der Lage ist, durch Zellen und Gewebe zu diffundieren und geringe Konzentrationen kleine Gradienten und damit einen geringen Massestrom der Substanz nach sich ziehen.

Eine Grundvoraussetzung, die oben geschilderten Probleme zu umgehen, besteht darin, dass die Substanzen in einer geeigneten Arzneiform so an den Applikationsort gebracht werden, dass sie dort möglichst homogen, d.h. gleichmäßig verteilt vorliegen. Dadurch wird gewährleistet, dass eine im Rahmen der Löslichkeit möglichst hohe Konzentration rasch und dauerhaft aufrecht erhalten wird.

Um dieses Ziel zu erreichen sind dem Fachmann zahlreiche Verfahren bekannt. Die einfachste Strategie ist das Lösen des Wirkstoffs in Ölen, wie z.B. flüssigen Triglyceriden, wie man sie in zahlreichen Pflanzenölen findet, oder wie sie als Neutralöl unter dem Handelsnamen Miglyol bekannt sind. Zwar können solche Lösungen am Auge und auch parenteral, d.h. z. B. in Form von Injektionen, angewendet werden, doch sie haben den Nachteil, dass sie den Arzneistoff nur schlecht ins wässrige Milieu freisetzen. Darüber hinaus können sich Probleme mit der Verträglichkeit mit dem Gewebe am Applikationsort ergeben. Die Applikation öliger Lösungen ist häufig von Entzündungsreaktionen begleitet. Im Rahmen der Anwendung am Auge kommt es teilweise zu einer Beeinträchtigung des Sehvermögens durch Schlierenbildung, die darauf zurückzuführen ist, dass sich das Öl mit der Tränenflüssigkeit nicht mischt. Darüber hinaus ist die Verweilzeit am Auge unzureichend.

Um die Verfügbarkeit des Arzneistoffs zu verbessern, bietet es sich speziell für Anwendungen am Auge an, Salben im Sinne des Arzneibuchs einzusetzen (Europäisches Arzneibuch 5.0, Halbfeste Zubereitungen zur kutanen Anwendung 5.0/0123 und Halbfeste Zubereitungen zur Anwendung am Auge 5.0/1163). Zwar ist deren Verweilzeit am Auge erhöht, allerdings erfordert deren sterile Herstellung einen aseptisch angelegten Produktionsvorgang, was die Kosten gegenüber einer Lösung, die sterilfiltriert werden kann, stark erhöht. Zudem gehen diese Zubereitungen mit einer sehr geringen Compliance einher, da der Patient Fremdkörpergefühl und eine substanzielle temporäre Sichtbehinderung durch Schlierenbildung erdulden muss. Die parenterale Applikation solcher Systeme scheitert darüber hinaus häufig an der Viskosität der Systeme, die eine Passage durch eine Kanüle verhindert.

"Lösungsvermittler" versuchen die Verwendung lipophiler Träger wie Öle oder teilweise auch Salben für lipophile Substanzen zu umgehen, indem die Substanz vom Lösungsvermittler umschlossen wird. Durch die dabei entstehende hydrophile Oberfläche dieser Aggregate, wird die Löslichkeit schlecht löslicher Substanzen in Wasser verbessert. Cyclodextrine und oberflächenaktive Substanzen, sog. Tenside, bieten beispielsweise diese Möglichkeit der Verbesserung der Löslichkeit. Daraus ergeben sich allerdings letztendlich Lösungen mit sehr kurzer Verweildauer am Auge und damit eine geringere Resorption.

Kolloidale Trägersysteme sind ein weiterer Versuch brauchbare Wirkstoffspiegel zu erhalten. Mizellare Formulierung bieten jedoch zu geringe Lagerstabilität und Haltbarkeit, liposomale Zubereitungen sind sehr kostspielig in Entwicklung und Herstellung. Beide Varianten können, wenn überhaupt nur bedingt steril filtriert werden und andere Sterilisationsverfahren wie Autoklavieren oder Strahlensterilisation würden die Arzneistoffe zu stark belasten, was auch hier eine kostspielige aseptische Herstellung unumgänglich macht.

Suspensionen bzw. Nanosuspensionen lipophiler Arzneistoffe bieten die Möglichkeit, den oben skizzierten Anforderungen an die Applikation gerecht zu werden, indem sie für eine "feine" und homogene Verteilung des Arzneistoffs am Applikationsort sorgen. Dem steht jedoch eine extrem aufwändige Herstellung gegenüber. Insbesondere bei Suspensionen kommt die geringe Lagerstabilität aufgrund von Agglomeration oder Sedimentation hinzu, die mit großem Aufwand so lange als möglich unterdrückt werden müssen.

Die bislang aussichtsreichste Möglichkeit lipophile Arzneistoffe zu verpacken und anzuwenden sind Emulsionen. Mit Restasis® ist in Amerika bereits ein Augentropfenpräparat auf dem Markt, welches effektive Wirkstoffspiegel bei mehrmaliger täglicher Applikation liefert. Doch auch hier stellen hohe Produktions- und Entwicklungskosten, sowie unzureichende Haltbarkeit und Stabilität des Systems bei Anwesenheit von Wasser ein Problem dar. Ein weiterer Nachteil ist, dass der Patient das Präparat, wie zum Beispiel im Fall von Restasis®, selbst für die Applikation vorbereiten und die Emulsion redispergieren muss, so dass der Therapieerfolg von der korrekten Vorbereitung und Anwendung des Patienten abhängt.

Wünschenswert ist daher eine Formulierung, welche die lipophilen Substanzen löst, aber dazu nicht auf ölige Lösungsmittel zurückgreift, die nicht mit Wasser mischbar sind. Nach Applikation sollte das System mit Wasser am Applikationsort so mischbar sein, dass es den Arzneistoff vollständig und örtlich gleichmäßig freisetzt. Weiter sollte das System kostengünstig zu produzieren sein, indem es z. B. als Lösung durch Filtration sterilfiltrierbar ist. Ein solches System würde in der Therapie einen hohen Anwendungskomfort gewährleisten, da es sowohl parenteral über Injektion oder am Auge durch Eintropfen ohne Schlierenbildung einfach und für den Organismus gut verträglich applizierbar wäre.

In Bezug auf eine gleichmäßige Verteilung eines Arzneistoffes an einem Applikationsort werden den oben genannten Anforderungen Suspensionen von Nanopartikel aus einer lipophilen Substanz mit einer Größe von kleiner 10 µm gerecht. Durch ihre große Oberfläche sorgen Nanopartikeln für eine hohe Lösegeschwindigkeit und damit verbesserte Bioverfügbarkeit der applizierten Substanz.

Abgesehen davon haben Nanosuspensionen jedoch gravierende Nachteile. Sie sind in der Herstellung aufwändig, die Verfahren verteuern die Entwicklung und Produktion solcher Systeme erheblich, und die Stabilität ist aufgrund von beispielsweise Aggregationsneigung und Partikelgrößenwachstum beschränkt.

Es war daher Aufgabe der vorliegenden Erfindung ein verbessertes System zur Verfügung zu stellen, das die vorstehend genannten Anforderungen erfüllt, mit dem die Bioverfügbarkeit von lipophilen Substanzen am oder im menschlichen oder tierischen Körper erhöht und erleichtert werden kann, und das auf einfache, kostensparende Weise, z. B. mittels Sterilfiltration, erhältlich ist.

Erfindungsgemäß wird diese Aufgabe gelöst durch eine pharmazeutische Zusammensetzung zur Verabreichung an das Auge, die mindestens ein mit Wasser mischbares, biokompatibles Lösungsmittel und mindestens einen darin gelösten lipophilen pharmazeutischen Wirkstoff enthält, wobei die pharmazeutische Zusammensetzung bei Kontakt mit wässriger Augenflüssigkeit eine Nanosuspension ausbildet, in der der mindestens eine lipophile pharmazeutische Wirkstoff in Form von Nanopartikeln suspendiert vorliegt.

Die vorliegende Erfindung greift auf die Vorteile einer Nanosuspension, d. h. der sehr fein dispergierten Teilchen zurück, entkoppelt diese aber von den vorstehend dargestellten Nachteilen, die Nanosuspensionen intrinsisch sind. Die erfindungsgemäße Zusammensetzung eignet sich damit hervorragend für die Verabreichung lipophiler Wirkstoffe an Mensch und Tier. Insbesondere eignet sich die erfindungsgemäße Zusammensetzung hervorragend für die Applikation lipophiler Wirkstoffe am Auge.

Die vorliegende Erfindung beruht auf dem Prinzip, dass die Zusammensetzung mit dem lipophilen Wirkstoff bei Kontakt mit Wasser den gelösten Wirkstoff unter Ausbildung definierter Nanostrukturen freisetzt, welche durch ihre sehr große Oberfläche und ihre gleichmäßige Verteilung verbesserte Rezeptionsbedingungen für schwer wasserlösliche Wirkstoffe bieten.

Hierzu wird der lipophile Wirkstoff mit einem Lösungsmittel, das mit Wasser mischbar ist, zu einer echten, d. h. insbesondere molekulardispersen, stabilen Lösung verarbeitet. Die Suspension des lipophilen Wirkstoffes bildet sich erst bei Einbringen dieser Lösung spontan am Applikationsort, indem Wasser, das als sogenanntes Antisolvens fungiert, der Mischung sozusagen das Lösungsmittel entzieht. Die erfindungsgemäße Zusammensetzung kann überall dort eingesetzt werden, wo eine wässrige Flüssigkeit vorhanden ist. Beispielsweise kann bei Applikation am Auge die in situ Präzipitation des Wirkstoffes durch Kontakt mit der Tränenflüssigkeit ausgelöst werden.

Mit der vorliegenden Erfindung ist es somit möglich, lipophile Wirkstoffe als Lösungen zu applizieren, ohne auf "ölige" Lösungsmittel oder andere lipophile Lösungsmittel wie Triglyceride oder Cholesterol zurückzugreifen. Bei diesen öligen oder lipophilen Lösungsmitteln handelt es sich um Lösungsmittel, die im Gegensatz zu dem erfindungsgemäß eingesetzten Lösungsmittel nicht mit Wasser mischbar sind, sondern bei Vermischung mit Wasser eine Emulsion, d. h. ein zweiphasiges System, ausbilden.

Erfindungsgemäß werden Lösungsmittel eingesetzt, die mit Wasser mischbar sind, und insbesondere ein homogenes System mit Wasser bilden.

Für die Erfindung geeignete Lösungsmittel können die lipophilen Wirkstoffe vollständig lösen, so dass eine echte Lösung entsteht. Vorzugsweise sollten die Lösungsmittel physiologisch verträglich und nicht toxisch sein. Insbesondere bevorzugt sollten sie transparent sein. Sie sollten flüssig sein und vorzugsweise einen Schmelzpunkt nahe oder unterhalb der Körpertemperatur von etwa 37 °C aufweisen.

Erfindungsgemäß eingesetzte Lösungsmittel sind Polyethylenglycole (PEG), Glycerol, Propylenglycol, Dimethylsulfoxid (DMSO), und N-Vinylpyrrolidon.

Es können auch Mischungen von zwei oder mehreren dieser Lösungsmittel eingesetzt werden.

Insbesondere bevorzugt sind Polyethylenglycole, Propylenglycol und N-Vinylpyrrolidon.

Als Lösungsmittel geeignete Polyethylenglycole bzw. deren Derivate weisen insbesondere eine Molmasse in einem Bereich von 50 bis 100.000 Da, vorzugsweise von 100 bis 1.000 Da und insbesondere bevorzugt von 200 bis 600 Da auf. Besonders bevorzugt sind Polyethylenglycole mit einer Molmasse, bei der die Polyethylenglycole im Bereich der Körpertemperatur von etwa 37 °C in flüssiger Form vorliegen.

Die höher molekularen festen Polyethylenglycole können z. B. in Mischung mit flüssigen Polyethylenglykolen oder anderen flüssigen Lösungsmitteln eingesetzt werden.

Die vorliegende Erfindung eignet sich prinzipiell für alle lipophile pharmazeutische Wirkstoffe wie Arzneistoffe. Es lassen sich damit erfolgreich lipophile, schwer bis praktisch in Wasser unlösliche Stoffe applizieren. Die vorliegende Erfindung eignet sich insbesondere auch für sehr schwer wasserlösliche bis praktisch wasserunlösliche Wirkstoffe. Beispiele hierfür sind Substanzen wie Cyclosporin A, Budesonid, Beclomethason, Triamcinolonacetonid, Dexamethason, Fluticasondipropionat.

Tritt die erfindungsgemäße Zusammensetzung mit dem gelösten Wirkstoff in Kontakt mit Wasser, kommt es zu Interaktionen, wobei sich das Lösungsmittel mit Wasser vermischt, und die dadurch bewirkte sinkende Löslichkeit für den Wirkstoff zum Ausfallen kleinster Feststoffteilchen des lipophilen Wirkstoffes führt.

Die Feststoffteilchen, d. h. die Nanopartikel, haben im Allgemeinen eine Größe im Bereich von bis zu 10 µm. Bevorzugt sind Partikel mit einer Größe in einem Bereich bis zu 1 µm und besonders bevorzugt Partikel mit einer Größe kleiner 1 µm. Partikel mit einer möglichst kleinen Teilchengröße sind für die erfindungsgemäße Anwendung besonders geeignet. So können beispielsweise Partikel mit einer Größe von wenigen Nanometern z. B. von bis zu 1 nm eingesetzt werden.

Die erhaltenen Partikel können eine unregelmäßige Oberfläche und Form aufweisen. Die Größe bezieht sich daher auf die Hauptachse der Partikel, d. h. auf die Achse mit der größten Erstreckung.

Das Vorliegen von Nanopartikeln in der Suspension und deren Bestimmung kann mittels an sich bekannter Verfahren geschehen, z. B. mittels Laser-Diffraktometrie und Photonen-Korrelationsspektroskopie.

Die Eigenschaften der erfindungsgemäßen Zusammensetzung sowie der Nanopartikel können je nach den Erfordernissen einer konkreten Anwendung auf vielfache Art und Weise variiert bzw. eingestellt werden. Dabei können auf Prinzipien und Mittel zurückgegriffen werden, die dem Galeniker für die Formulierung von flüssigen Arzneistoffen an sich bekannt sind.

So kann die Ausfällung der Nanopartikel aus der Lösung durch Einbringen von einem oder mehreren Tensiden (oberflächeaktive Substanzen) gesteuert werden. Vorzugsweise werden für die vorliegende Erfindung physiologisch verträgliche Tenside eingesetzt. Beispiele hierfür sind Cetylalkohol, Stearylalkohol, Natriumcetylstearylsulfat, Glycerolmonostearat und Sorbitanfettsäureester. Besonders bevorzugt werden Tenside eingesetzt, wie sie im physiologischen Milieu des menschlichen und tierischen Organismus vorkommen, wie z. B. Lecithin, Cholesterol, Phosphatidylcholin, Phosphatidylserin, Phosphatidylinositol und Phosphatidylethanolamin.

Es können hierzu auch die Lösungseigenschaften des Lösungsmittels durch Zusatz eines Cosolvens variiert werden, wobei sich je nach Kombination eine Änderung der Teilchengröße erzielen lässt.

Es können auch amphiphile Phospholipide wie z. B. Diheptanoylglycerophosphocholin (DHGPC), ein physiologisch nicht vorkommendes Phosphatidylcholin eingesetzt werden, die auch als Cosolvens wirken können.

Eine Möglichkeit die Partikelgröße zu steuern besteht darin, das Partikelwachstum über die physikochemischen Eigenschaften der erfindungsgemäßen Lösung mit der darin gelösten Substanz zu beeinflussen.

Eine dieser Eigenschaften ist die Viskosität. Durch Erhöhung der Viskosität des Systems lässt sich eine diffusionsbegrentzte Aggregation der Partikel erzielen. Dabei entstehen fraktale Strukturen, d. h. Teilchen mit hoher Oberflächenunregelmäßigkeit, die sich daher durch eine besonders große Oberfläche auszeichnen.

Für die Erhöhung der Viskosität können der erfindungsgemäßen Zusammensetzung beispielsweise viskositätserhöhende Mittel zugesetzt werden, die dem Galeniker für die parenterale Applikation bekannt sind. Beispiele hierfür sind viskositätserhöhende Polymere sowie Polyethylenglykol, Cellluloseether, Polyvinylpyrrolidon und Polyvinylalkohol.

Einige Substanzen können mit den Nanopartikeln Einschlussverbindungen ausbilden, indem sie die Nanopartikel umgeben. Auch durch die Ausbildung derartiger Einschlussverbindungen lässt sich die Partikelgröße einstellen, da dadurch ein Wachstum sowie die Agglomeration der Partikel verhindert wird. Ein Beispiel für eine derartige Substanz ist Polyvinylpyrrolidon, das gleichzeitig als viskositätserhöhendes Mittel wirkt.

Je nach Anwendung oder Lösungsmittelsystem kann es vorteilhaft sein, wenn die entstehenden Nanopartikel möglichst lange am Freisetzungsort verharren, um die Resorptionsquote zu steigern. Dies kann beispielsweise durch Verminderung der Beweglichkeit der Nanopartikel erreicht werden.

Die Beweglichkeit der Nanopartikel kann beispielsweise durch Zusatz von viskositätserhöhenden Mitteln oder durch Optimierung der elektrostatischen Anziehungskräfte bewirkt werden.

Zur Erhöhung der Viskosität können beispielsweise die vorstehend genannten viskositätserhöhenden Mittel eingesetzt werden.

Zur Verringerung der Beweglichkeit der Nanoteilchen kann die elektrostatische Wechselwirkung der Nanoteilchen mit dem Gewebe am Applikationsort ausgenutzt werden.

Beispielsweise kann das Zetapotential der Nanopartikel gegensinnig zu dem Potential des Gewebes eingestellt werden. Das gegensinnige Potential kann eine Substanzeigenschaft der Nanopartikel an sich sein. Es können Zusätze zur Einstellung des Zetapotentials zugesetzt werden, wie z. B. Phosphate oder Citrate. Derartige Zusätze zur Einstellung des Zetapotentials sind dem Fachmann beispielsweise als Peptisatoren bekannt.

Basierend auf den vorstehenden Prinzipien können unterschiedlichste Zusammensetzungen des erfindungsgemäßen Lösungsmittelsystems erhalten werden.

Nachstehend werden Beispiele für unterschiedliche Ausführungsformen aufgeführt, anhand derer aufgezeigt wird, wie durch Kombination von ein oder mehreren Zusätzen und Hilfsstoffen die Eigenschaften des erfindungsgemäßen Lösungsmittelsystems bzw. der Nanopartikel variiert werden können.
1. Die einfachste Variante ist eine binäre Formulierung eines erfindungsgemäß geeigneten Lösungsmittels mit einer lipophilen Substanz. Durch Beimengung eines Cosolvens können die Lösungseigenschaften variiert werden, wobei in Abhängigkeit der jeweiligen Kombination gewünschte Partikelgrößen erzielt werden können.
2. Mehr Möglichkeiten zur Beeinflussung und Kontrolle der Entstehung, Größe und Funktionalität der Nano-Partikel lassen sich mit ternären Systemen erhalten, die zusätzlich zu dem Lösungsmittel beziehungsweise Lösungsmittelgemisch und der lipophilen Substanz weitere Zusätze oder Hilfsmittel enthalten.
   a) Durch Zusatz von Tensiden oder anderen amphiphilen Substanzen kann der Wachstumsprozess der Nanopartikel beeinflusst werden.
   b) Durch Zusatz viskositätserhöhender Mittel lässt sich das Teilchenwachstum und die Diffusion der ausgefällten Nanopartikel verlangsamen.
      Zur Erhöhung der Viskosität können auch in situ gelierende Substanzen eingesetzt werden. Bei den "in situ" gelierenden Substanzen erfolgt die Gelierung erst am Anwendungsort durch Änderung der Umgebungsbedingungen, wie des pH-Wertes, der Temperatur, der Scherbelastung oder anderen äußeren Einflüssen.
   c) Durch Variation des Zetapotentials lässt sich der Abtransport der Nanopartikel verlangsamen. Vorzugsweise können hierzu Mittel zur Einstellung des Zetapotentials eingesetzt werden, die das Zetapotential der Nanopartikel positiv einstellen können.
   d) Eine Funktionalisierung der Nanopartikel beziehungsweise des gesamten Systems der vorstehend unter 2a bis c) beschriebenen Formulierungen lässt sich durch geeignete Wahl der amphiphilen beziehungsweise viskositätserhöhenden Mittel erzielen.
      i) So können bei Einsatz geladener Tenside die von ihnen eingeschlossenen Nanopartikel in Abhängigkeit der Ladung längere Zeit an entgegengesetzt geladenen Oberflächen fixiert werden.
      ii) Durch den Einsatz geladener viskositätserhöhender Mittel oder in situ gelierender Substanzen lässt sich das gesamte System für bestimmte Intervalle an entgegengesetzt geladenen Oberflächen zurückhalten.
3. Ausgehend von den vorstehend genannten Ausführungsformen zu 1. und 2. lassen sich durch quaternäre Formulierungen noch mehr Variablen in das System und damit eine erhöhte Spezifität und Effektivität einbringen.
   a) So können zu einer Lösung gemäß 1) zwei verschiedene amphiphile Substanzen gegeben werden. Je nach Kombination und Eigenschaften dieser amphiphilen Substanzen wie Ladung, Größe des lipophilen Teils etc. lassen sich die für die Tenside gemäß 2a) beschriebenen Effekte verstärken beziehungsweise optimieren.
   b) Die Anwesenheit eines Tensids in der Ausführungsform gemäß 2a) erlaubt das Einarbeiten kleiner Anteile an Wasser in die Formulierung, wodurch die spontane Ausfällreaktion bei Kontakt mit Wasser am Anwendungsort unterstützt wird.
   c) Eine hohe Effektivität kann erzielt werden durch Zusatz einer Kombination von viskositätserhöhenden Mitteln und Tensiden zu der Ausführungsform gemäß 1. Die Nanopartikel können hier, geladen oder ungeladen, feinverteilt in einem gelartigen Zustand eingebunden länger an Oberflächen verweilen und den Wirkstoff effektiver abgeben. Zur Viskositätserhöhung kann ein viskositätserhöhendes Mittel zugesetzt werden oder eine in situ gelierende Substanz.

Die vorstehend aufgeführten Ausführungsformen verstehen sich lediglich als Beispiele zur Veranschaulichung der vielfältigen Variationsmöglichkeiten, die die erfindungsgemäße Zusammensetzung bietet.

Selbstverständlich können der grundlegenden Ausführungsform gemäß 1. die vorstehend genannten Zusätze sowie weitere dem Galeniker geläufige Zusätze je nach Bedarf in beliebiger Kombination zugesetzt werden.

Beim erfindungsgemäßen Einsatz der erfindungsgemäßen Zusammensetzung entsteht bei Kontakt mit wässrigen Flüssigkeiten ein Präzipitat des zuvor gelösten Wirkstoffes in Form von Nanopartikeln, da die Löslichkeit des Wirkstoffes beim Durchmischen von Lösungsmitteln mit Wasser herabgesetzt wird. Die Partikelgröße kann entweder durch die dabei "aktiv" werdenden amphiphilen Zusätze, durch viskositätserhöhende Zusätze oder eine Kombination davon möglichst klein gehalten und Agglomerate verhindert werden.

Die vorliegenden Erfindung betrifft damit eine in situ entstehende Nanosuspension mit verschieden einstellbaren Funktionalitäten der entstehenden Nanopartikel und vereint so die Vorteile einer echten Lösung mit den Vorteilen einer Nanosuspension, ohne dabei die Nachteile der beiden mit sich zu bringen. Die Erfindung stellt damit ein kostengünstig herzustellendes, steril filtrierbares Produkt mit verbesserter Applikation und Resorption dar, wodurch eine höhere Bioverfügbarkeit erzielt wird.

Die erfindungsgemäße Zusammensetzung eignet sich insbesondere als eine pharmazeutische Zusammensetzung zur Verabreichung eines pharmazeutischen Wirkstoffes am Auge.

Es versteht sich, dass bei Bedarf für die Herstellung einer pharmazeutischen Zusammensetzung auf Basis der erfindungsgemäßen Lösung die pharmazeutische Zusammensetzung zusätzlich weitere Hilfsstoffe enthalten kann, wie sie dem Galeniker für die jeweilige Anwendung geläufig sind.

Nachstehend wird das Prinzip der vorliegenden Erfindung sowie die Herstellung der erfindungsgemäßen Lösung anhand von Beispielen weiter veranschaulicht.

### Beispiele

### Beispiel 1: Mischbarkeit von Lösungsmittel und Wasser

Polyethylenglycol 400, ein klares, flüssiges Lösungsmittel wurde in verschiedenen Verhältnissen von 1:10 bis 1:1 (H₂O : PEG 400) mit Wasser gemischt und die resultierende Lösung charakterisiert. Durchmischt wurde die Lösung auf einem Vortex Mischer bei Raumtemperatur (21°C). Alle untersuchten Verhältnisse ergaben ein transparentes, homogenes Gemisch.

### Beispiel 2: Lösung aus Wirkstoff und Lösungsmittel

Als Lösungsmittel diente Polyethylenglycol 400. In 1ml dieser klaren, bereits etwas viskoseren Flüssigkeit wurden 20mg Cyclosporin A gelöst (c(CsA) = 20mg/ml). Das Lösen geschah bei Raumtemperatur (21 °C) unter Schütteln auf einem VortexMischer. Die resultierende Lösung war transparent und frei von Schwebstoffteilchen. Dies wurde unter dem Mikroskop bestätigt. Bei Zugabe von 100µl Wasser zu 500µl dieser Lösung bildete sich spontan eine Trübung. Unter dem Mikroskop betrachtet konnte man ein grob strukturiertes Präzipitat mit deutlichen Kristallen erkennen.

### Beispiel 3: Lösung aus Wirkstoff, Regulatoren und Lösungsmittel

In 1 ml Polyethylenglycol 400 wurden auf dem Vortex Mischer 20 mg Phospholipid (Diheptanoylglycerophosphocholin (DHGPC)) gelöst. Dies ergab bei Raumtemperatur (21 °C) eine klare Lösung. Danach wurden darin 20mg Cyclosporin A und gleichen Bedingungen gelöst. Das resultierende Gemisch war transparent und frei von Schwebstoffteilchen. Die mikroskpoischen Aufnahmen belegten dies. Das Zutropfen von 100µl Wasser zu 500µl dieser Lösung bewirkte eine spontane Präzipitation. Unter dem Mikroskop betrachtet konnte man erkennen, dass sich fein verteilte Strukturen ohne erkennbare, größere Kristalle formiert hatten.

### Beispiel 4: Aufnahmefähigkeit für Wasser

Es wurde eine Lösung von 10mg Phospholipid und 2mg Cyclosporin A in 1ml Polyethylenglycol 400 hergestellt. Das Lösen der Substanzen geschah bei Raumtemperatur (21 °C) auf einem Vortexmischer. Zu dieser Lösung wurde Wasser in Verhältnissen von 1:10 bis 3,5:10 (H₂O : Lösung) zugetropft und es wurde gemischt. Initial trübte sich das System. Diese Trübung verschwand kurz darauf bei allen Verhältnissen bis 3:10 (H₂O : Lösung) und es entstand eine transparente Lösung, frei von Schwebstoffteilchen. Unter dem Mikroskop konnte man sehr fein dispergierte, offensichtlich von einer Phospholipidschicht abgeschirmte Partikel erkennen. Die Mischungen mit einem höheren Wasseranteil klarten nicht auf und ein dauerhaftes Präzipitat bildete sich.

## Patentansprüche

1. Pharmazeutische Zusammensetzung zur Verabreichung an das Auge enthaltend mindestens ein mit Wasser mischbares biokompatibles Lösungsmittel und einen darin gelösten lipophilen pharmazeutischen Wirkstoff, wobei die pharmazeutische Zusammensetzung bei Kontakt mit wässriger Augenflüssigkeit eine Nanosuspension ausbildet, in der der lipophile pharmazeutische Wirkstoff in Form von Nanopartikeln suspendiert vorliegt, wobei das mit Wasser mischbare biokompatible Lösungsmittel ausgewählt ist unter einem Polyethylenglycol, Glycerol, Propylenglycol, Dimethylsulfoxid, N-Vinylpyrrolidon und Mischungen davon.

2. Pharmazeutische Zusammensetzung nach Anspruch 1,
**wobei** die Lösung zusätzlich mindestens einen Zusatzstoff ausgewählt unter einem Tensid, einem viskositätserhöhenden Zusatz und Mittel zur Einstellung des Zetapotentials der Nanopartikel enthält.

3. Pharmazeutische Zusammensetzung nach Anspruch 1 oder 2,
**wobei** die Lösung zwei oder mehrere lipophile pharmazeutische Wirkstoffe enthält.

4. Pharmazeutische Zusammensetzung nach einem der vorhergehenden Ansprüche,
**wobei** die Nanopartikel kleiner als 10 µm (bezogen auf die Hauptachse) sind.

5. Pharmazeutische Zusammensetzung nach einem der vorhergehenden Ansprüche,
**wobei** die Nanopartikel eine Größe von 1 nm oder mehr (bezogen auf die Hauptachse) aufweisen.

6. Verwendung einer pharmazeutischen Zusammensetzung nach einem der Ansprüche 1 bis 5 zur Herstellung eines Medikaments zur Verabreichung eines oder mehreren lipophilen pharmazeutischen Wirkstoffen an ein Auge eines Menschen oder eines Tieres.

## Claims

1. A pharmaceutical composition for application to the eye comprising at least one water-miscible biocompatible solvent and a lipophilic pharmaceutical substance dissolved therein,
**wherein** the pharmaceutical composition forms a nanosuspension upon contact with an aqueous eye fluid, the lipophilic pharmaceutical substance therein being present in the form of nanoparticles in suspension,
**wherein** the water-miscible biocompatible solvent is selected from the group consisting of polyethylene glycol, glycerol, propylene glycol, dimethylsulfoxid and N-vinylpyrrolidone and a mixture thereof.

2. The pharmaceutical composition according to claim 1,
**wherein** the solution additionally contains at least an additive selected from the group consisting of a surfactant, a viscosity-enhancing additive, and means for adjusting the zeta potential of the nanoparticles.

3. The pharmaceutical composition according to claim 1 or 2,
**wherein** the solution includes two or more lipophilic pharmaceutical substances.

4. The pharmaceutical composition according to any of the proceeding claims,
**wherein** the nanoparticles are smaller than 10 µm (with respect to the main axis).

5. The pharmaceutical composition according to any of the proceeding claims,
**wherein** the nanoparticles have a size of 1 nm or more (in respect to the main axis).

6. Use of the pharmaceutical composition according to any of the claims 1 to 5 for manufacturing a medicine for application of one or more lipophilic pharmaceutical substances to the eye of a human or an animal.

## Revendications

1. Composition pharmaceutique destinée à être administrée dans l'oeil contenant au moins un solvant biocompatible pouvant être mélangé avec de l'eau et un principe actif pharmaceutique lipophile dissous dans celui-ci, **dans** laquelle la composition pharmaceutique réalise, en contact avec un fluide oculaire aqueux, une nanosuspension,
dans laquelle le principe actif pharmaceutique lipophile est présent en suspension sous la forme de nanoparticules,
**dans** laquelle le solvant biocompatible pouvant être mélangé avec de l'eau est choisi parmi un polyéthylène glycol, glycérol, propylène glycol, diméthylsulfoxyde, N-vinylpyrrolidone et des mélanges de ceux-ci.

2. Composition pharmaceutique selon la revendication 1,
**dans** laquelle la solution contient en plus au moins un additif choisi parmi un tensioactif, un additif d'augmentation de la viscosité et des agents servant à régler le potentiel zêta des nanoparticules.

3. Composition pharmaceutique selon la revendication 1 ou 2,
**dans** laquelle la solution contient deux ou plusieurs principes actifs pharmaceutiques lipophiles.

4. Composition pharmaceutique selon l'une quelconque des revendications précédentes,
dans laquelle les nanoparticules sont inférieures à 10 µm (par rapport à l'axe principal).

5. Composition pharmaceutique selon l'une quelconque des revendications précédentes,
**dans** laquelle les nanoparticules présentent une taille de 1 nm ou plus (par rapport à l'axe principal).

6. Utilisation d'une composition pharmaceutique selon l'une quelconque des revendications 1 à 5 servant à fabriquer un médicament servant à administrer un ou plusieurs principes actifs pharmaceutiques lipophiles dans un oeil d'un être humain ou d'un animal.
